# EUROPÄISCHE PATENTSCHRIFT

(11) **EP 3 106 849 B1**
(45) Veröffentlichungstag und Bekanntmachung des Hinweises auf die Patenterteilung: **23.05.2018**
(21) Anmeldenummer: 15172551.2
(22) Anmeldetag: 17.06.2015
(51) Int. Cl.: G01J 1/42, A61C 13/15

(54) **LICHTHÄRTGERÄT**
LIGHT HARDENING DEVICE
APPAREIL DE DURCISSEMENT À LA LUMIÈRE

(43) Veröffentlichungstag der Anmeldung: 21.12.2016
(60) Teilanmeldung: 17210443.2
(73) Patentinhaber: Ivoclar Vivadent AG, 9494 Schaan (LI)
(72) Erfinder: Senn, Bruno, 9470 Buchs (CH); Tommasini, Dario, 7302 Mastrils (CH)
(74) Vertreter: Baronetzky, Klaus

(56) Entgegenhaltungen:
- EP-A1- 1 236 444
- DE-A1-102014 224 026
- JP-B2- 2 992 175
- US-B1- 6 485 301

## Beschreibung

Die Erfindung betrifft ein Lichthärtgerät, gemäß dem Oberbegriff von Anspruch 1.

Es ist seit langem bekannt, dass für die Gewährleistung einer zuverlässigen Polymerisation oder Härtung von Dentalmaterialien wesentlich ist, dass eine exakt vorgegebene Lichtmenge zugeführt werden muss. Die Soll-Lichtmenge hängt von vielen Faktoren ab, beispielsweise von der Form und der Schichtstärke sowie der Masse der zu härtenden Dentalrestauration, dem Photoinitiator, der die Polymerisation triggert, also auslöst, dem emittierten Wellenlängenbereich des Lichthärtgeräts an dessen Lichtleitstab, indirekt aber auch beispielweise von der Lichtquelle selbst, die eine Degradation erfahren kann, oder auch der Qualität des Lichtleitstabs, der beispielsweise auch gebrochen sein kann - ohne dass der Benutzer von etwaigen Rissen Kenntniss hat.

Es ist andererseits seit langem bekannt, dass man die Lichtquelle kalibrieren sollte, um die Lichtabgabe zu vergleichmäßigen. Als eine der zahlreichen Beispiele hierfür sei auf die DE 196 36 266 A1 verwiesen, gemäß welcher die dortige Lichtquelle von einem Detektor zur Messung der Lichtintensität in der Vorrichtung kontrolliert werden soll (vergleiche Spalte 3, Zeile 48 ff).

Nachteilig hierbei ist, dass der Lichtleitstab nicht in die Kalibration einbezogen werden kann.

Es sind aber auch bereits insofern verbesserte Lösungen bekannt geworden, die den Lichtleitstab einbeziehen. Hierzu sei auf die DE 10 2007 052 643 A1 verwiesen, die einen Lichtsensor an der Basisstation aufweist. Das Handstück muss bei dieser Lösung mit dem Ende des Lichtleitstabs auf die Sensorfläche gehalten werden, und es soll so die Lichtemission gemessen werden, insbesondere auch die Lichtintensität.

Wenn mehrere - gleichartige - Lichthärtgeräte verwendet werden, besteht die Möglichkeit, dass Basisstation und Handstück vom Zahnarzt nicht richtig zugeordnet werden, und die Messung der abgegebenen Lichtintensität sich dann auf das falsche Lichthärtgerät bezieht. Dies gilt im besonderen Maße, wenn die Übertragung der Kalibrierungsinformation lediglich dann erfolgt, wenn das Lichthärtgerät eingesteckt ist, wie es bei der Lösung gemäß der DE 10 2007 052 643 A1 der Fall ist, bei der die gemessene Lichtleistung dann in der Basisstation zwischengespeichert werden muss.

Ferner wäre die noch zuverlässigere Erkennung eines Mikrobruchs in dem Lichtleitstab wünschenswert.

EP 1236444 A1 offenbart eine Einrichtung zur Polymerisation von lichthärtenden Kunststoffen, insbesondere von zahnärztlichen Füll- oder Klebematerialien, enthaltend mindestens eine. Lichtquelle zur Erzeugung einerseits eines Polymerisationslichtes in einem zur Polymerisation geeigneten Lichtspektrum und andererseits eines Pilotlichtes, eine Energieversorgungsquelle zur Speisung der Lichtquelle, ein sich an die Lichtquelle anschließendes, Lichtleitsystem mit Lichtleitern einerseits zur Übertragung des Polymerisationslichtes und des Pilotlichtes auf das zu bearbeitende Objekt, und andererseits zur Einkopplung des am Objekt reflektierten Pilotlichtes in einen Lichtsensor, enthaltend ferner eine elektronische Steuereinheit in welcher die vom Lichtsensor ausgehenden Signale verarbeitet werden und welche der Lichtquelle für das Polymerisationslicht und einem die Einschaltdauer der Lichtquelle bestimmenden Timer ein Startsignal geben, wobei ein Verstärker vorgesehen ist der die vom Lichtsensor ausgehenden Signale verstärkt und dass die Ansprechschwelle des Verstärkers so eingestellt ist, dass die Lichtquelle für das Polymerisationslicht erst bei Erreichen eines definierten Abstandes eingeschaltet wird. Weiterhin ist dem einen Ende des Lichtleiters, der das Lichtaustrittsfenster enthält, ein aufsetzbarer Messtubus mit Stirnfläche, die das Licht total reflektiert, zugeordnet, mit dem das optische System bzw. die Leistungsfähigkeit des Systems überprüft werden kann. Daher liegt der Erfindung die Aufgabe zugrunde, ein Lichthärtgerät für das Härten von Dentalmaterialien gemäß dem Oberbegriff von Anspruch 1 zu schaffen, das hinsichtlich der Erkennungsleistung und der Zuverlässigkeit noch weiter verbessert ist, wobei die Kosten für die Realisierung sich höchstens im Rahmen der bereits bekannten Lösungen bewegen sollen.

Diese Aufgabe wird erfindungsgemäß durch Anspruch 1 gelöst. Vorteilhafte Weiterbildungen ergeben sich aus den Unteransprüchen.

Überraschend lässt sich mit der erfindungsgemäßen Lösung, einen preisgünstigen Spiegel zu verwenden, dessen reflektiertes Licht erneut durch den Lichtleitstab hindurchgeführt wird, die Erkennbarkeit von Mikrorissen deutlich verbessern. Die Mikrorisse streuen regelmäßig das eingeleitete Licht und erhöhen insofern die Dämpfung durch den Lichtleitstab; durch die erfindungsgemäße Verdopplung der Dämpfung gegenüber dem ordnungsgemäßen Betrieb des Lichtleitstabs bei der Kalibration lässt sich die Erkennungsrate deutlich verbessern.

Dadurch, dass die Steuervorrichtung dem Handstück unmittelbar zugeordnet ist, ist eine Verwechslung bzw. eine Fehlzuordnung der ermittelten Daten unmöglich; überraschend lässt sich daher dieses im Stand der Technik vorhandene Problem mit ausgesprochen einfachen Maßnahmen und dementsprechend preisgünstig sicher vermeiden.

Erfindungsgemäß lässt sich nun von dem Lichteinfall auf den Sensor, der sich in dem Handstück befindet, ein Kalibrierprogramm auslösen. Hierfür ist es bevorzugt, dass ein Schwellwert eingerichtet wird, damit nicht von der Reflexion an beispielsweise einer Zahnoberfläche bereits das Kalibrierprogramm gestartet wird.

Alternativ ist es auch möglich, das Kalibrierprogramm zu starten, indem ein Mikroschalter betätigt wird, der dann ausgelöst wird, wenn das Ende des Lichtleitstabs in einer vorgegebenen Position bezogen auf den erfindungsgemäßen Spiegel ausgerichtet ist. Eine dritte Möglichkeit besteht darin, das Kalibrierprogramm dann auszulösen, wenn der Benutzer dies wünscht.

Erfindungsgemäß ist der Spiegel an der Basisstation angebracht. Eine Ausrichthilfe ist realisiert, die gewährleistet, dass sich der Spiegel im Kalibriermodus exakt senkrecht zu der Lichtaustrittsfläche des Lichtleitstabs erstreckt. Die Ausrichthilfe kann beispielsweise durch eine Hülse realisiert sein, in die der Lichtleitstab mit seinem vorderen Ende einsteckbar ist und die dieses in einem Winkel von 90° also senkrecht zu dem Spiegel hält. Auch diese Ausgestaltung gewährleistet, dass stets die gleichen Kalibrierbedingungen im Kalibriermodus vorliegen.

Der Sensor ist in dem Handstück so angeordnet, dass er die reflektierte Strahlung, die den Lichtleitstab erneut durchtritt, erfasst. Bevorzugt ist er optisch entkoppelt von der Lichtquelle angebracht, beispielsweise im Strahlengang dieser hinter dieser und er erhält über optische Koppelelemente die reflektierte Strahlung des Spiegels, nachdem sie den Lichtleitstab durchtreten hat.

In vorteilhafter Ausgestaltung ist es vorgesehen, dass dem Benutzer des Lichthärtgeräts die Qualität und aktuelle Leistung des Lichthärtgeräts des Kalibriermodus in geeigneter Weise angezeigt wird, sei es durch Farbanzeigen wie rot, gelb, grün oder als numerischer Wert unter Angabe der Lichtleistung. Der Benutzer kann so frühzeitig eine Degradation eines Lichthärtgeräts feststellen und beispielsweise dafür sorgen, dass Verschmutzungen die Lichtleistungen nicht beeinträchtigen.

Es ist auch möglich, mit einer entsprechenden Anzeige den Benutzer zur Reinigung oder zum Austausch des Lichtleiters aufzufordern.

Steckbare Lichtleiter sind im Grunde aufgrund der Autoklavierbarkeit bevorzugt. Sie bergen jedoch die Gefahr, dass, wenn der Lichtleitstab versehentlich nach der Reinigung nicht erneut eingesteckt wird, das Linsensystem des Lichthärtgeräts verschmutzt, beispielsweise verstaubt. Dies führt ebenfalls zur Degradation der Lichtleistung, wobei es aber möglich ist, dass der Benutzer gemäß Betriebsanleitung des Lichthärtgeräts auch hier selbst eine Reinigung vornimmt, beispielsweise mit einem weichen Pinsel oder ähnlichem die Optik reinigt.

Der Lichtleitstab selbst kann insbesondere an seinem vorderen Ende, das häufig Kontakt mit dem Mund des Patienten hat, Ablagerungen tragen, die durch Autoklavieren nicht oder nicht vollständig beseitigt werden. Hier kann dem Benutzer eine Reinigung dieses vorderen Endes per Anzeige empfohlen werden, so dass ein vorzeitiger Austausch des im übrigen noch funktionsfähigen Lichtleitstabs vermieden werden kann.

Andererseits können Lichtleitstäbe Mikrorisse aufweisen, also Risse, die von außen nicht ohne weiteres zu erkennen sind - wenn nicht die typischerweise schwarze Kunststoffschutzhülle entfernt wird. Der Lichtleitstab ist in diesem Zustand typischerweise nicht vollständig gebrochen, sondern weist lediglich Risse auf, die beispielsweise durch eine unsaubere Handhabung, durch Fallenlassen oder dergleichen hervorgerufen worden sind.

Diese Mikrorisse führen zur Erhöhung der Dämpfung der Durchlässigkeit des Lichtleitstabs. Bei einem typischen Riss ist die Lichtleistung im Betrieb dann beispielsweise auf den Faktor 0,8 abgesenkt. Erfindungsgemäß ist die Signifikanz dieser Verschlechterung im Kalibriermodus jedoch wesentlich erhöht; die reflektierte Strahlung wird erneut um den Faktor 0,8 reduziert, so dass sich insgesamt eine Reduktion um 0,8 mal 0,8, also 0,64, ergibt, die wesentlich besser von dem Sensor und der zugehörigen Steuereinrichtung des Lichthärtgeräts erkennbar ist.

Erfindungsgemäß ist es vorgesehen, im Kalibriermodus unkontinuierliches Licht von der Lichtquelle austrahlen zu lassen, beispielsweise sinusförmiges oder gepulstes Licht. Dies ermöglicht die Unterscheidung zwischen Umgebungslicht und dem emittierten Licht der Lichtquelle. Hierbei wird von der Steuervorrichtung dann kurzerhand die Amplitude des empfangenen Ausgangssignals des Sensors ausgewertet und als Nutzlicht für die Kalibrierung verwendet.

Innerhalb des Regelbereichs lässt sich im Kalibriermodus auch eine Selbstnachkalibration realisieren; beispielsweise kann eine Erhöhung oder Erniedrigung der Lichtleistung um plus/minus 20% vorgegeben sein.

Erfindungsgemäß günstig ist es auch, wenn das Handstück des Lichthärtgeräts die Entnahme des Lichtleiters automatisch erkennt und dann umgehend den Kalibriermodus anfordert bzw. beim nächsten Einschalten hierzu auffordert.

Dies ermöglicht die automatische Kompensation der Exemplarstreuung von Lichtleitstäben beim Tausch der Lichtleitstäbe. Zudem wird der Tatsache damit Rechnung getragen, dass beim Reinigen des Lichtleitstabs die Durchlässigkeit meist verbessert ist, so dass insofern eine Kalibration empfehlenswert ist.

Bevorzugt sind der oder die Sensoren etwas außerhalb der Zentralachse des rückwärtigen Endes des Lichtleitstabs angeordnet. Sie erfassen damit bevorzugt das am Außenumfang des Lichtleitstabs übertragene reflektierte Licht, während die Lichtquelle bevorzugt den zentralen Bereich des Lichtleitstabs beaufschlagt.

Weitere Vorteile, Einzelheiten und Merkmale ergeben sich aus der nachfolgenden Beschreibung eines Ausführungsbeispiels der Erfindung anhand der Zeichnungen.

Es zeigen:
- Fig. 1: eine schematische Ansicht einer Ausführungsform eines erfindungsgemäßen Lichthärtgeräts in perspektivischer Darstellung;
- Fig. 2: die Draufsicht auf die Ausführungsform gemäß Fig. 1; und
- Fig. 3: eine leicht schmatisierte Ansicht einer weiterer Ausführungsform eines Lichthärtgeräts, welche nicht Teil der vorliegenden Erfindung ist.

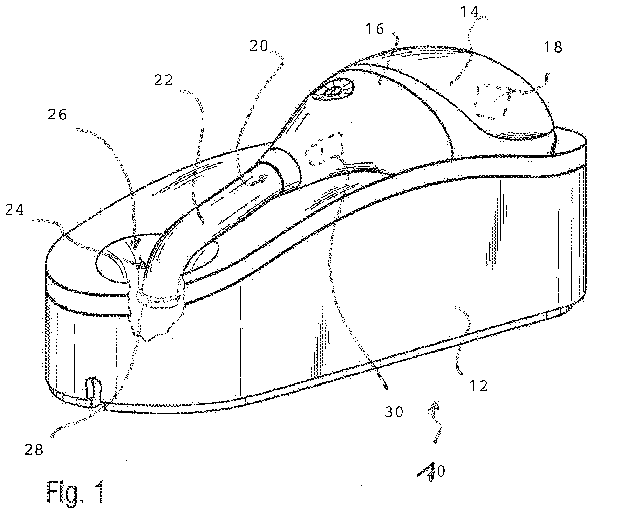

Das in Fig. 1 dargestellte Lichthärtgerät 10 weist eine Basisstation 12 und ein Handstück 14 auf, die in an sich bekannter Weise voneinander trennbar sind. Die Basisstation 12 bietet in dem dargestellten Ausführungsbeispiel eine räumlich fixierte Auflage bzw. Einlage für das Handstück 14, und zudem die Möglichkeit, den Akkumulator des Handstücks 14 automatisch aufzuladen.

Ferner ist eine bevorzugt drahtlose Datenübertragung zwischen Handstück 14 und Basisstation 12 vorgesehen. Diese dient beispielsweise der Anpassung des Ladestroms an die Erfordernisse. Eine bidirektionale Kommunikation ist optional auch möglich, beispielsweise, wenn Daten über den Betrieb des Handstücks in der Zahnarztpraxis, die beispielsweise mehrere erfindungsgemäße Lichthärtgeräte haben kann, an eine Zentrale übetragen werden sollen.

Das Handstück 14 weist ein Gehäuse 16 auf, das eine schematisch dargestellte Steuervorrichtung 18 aufnimmt. Das Gehäuse 16 weist an seinem vorderen Ende eine Aufnahme 20 für einen Lichtleitstab 22 auf. In dem dargestellten Ausführungsbeispiel ist der Lichtleitstab 22 austauschbar und rastet in der Aufnahme 20 ein; in einer anderen Ausführungsform ist er fest mit dem Gehäuse 16 verbunden.

In an sich bekannter Weise ist der Lichtleitstab 22 abgekröpft, und zwar vor seinem vorderen Ende 24. Dieses liegt in der dargestellten Ruheposition in einer Vertiefung 26 der Basisstation 12, wobei das Lichtaustrittsende des Lichtleitstabs 22 in die Vertiefung 26 hineinragt.

Erfindungsgemäß weist die Basisstation 12 unten in der Vertiefung 26 einen Spiegel 28 auf, der genau zu dem vorderen Ende 24 des Lichtleitstabs 22 passt und gegenüber diesem exakt ausgerichtet ist, wenn das Handstück 14 in der Ruheposition in der Basisstation 12 liegt.

Der Spiegel ist leicht schräg gegenüber der Horizontalen ausgerichtet, entsprechend der Lichtaustrittsfläche des Lichtleitstabs und weist im sauberen Zustand einen Reflektionswert von mehr als 95% auf. Hierdurch wird das aus dem Lichtleitstab 22 austretende Licht nahezu vollständig reflektiert und in den Lichtleitstab 22 zurückgeworfen.

Dem hinteren Ende des Lichtleitstabs 22 und der Aufnahme 20 benachbart ist ein Sensor 30 in dem Handstück 14 vorgesehen, der das von dem Spiegel 28 zurückgeworfene und erneut durch den Lichtleitstab 22 hindurchgeführte Licht erfasst und dessen Ausgangssignal der Steuervorrichtung 18 zugeleitet wird.

Die Steuervorrichtung 18 weist nun ein spezielles Kalibrierprogramm auf, das bei einem bestimmten Lichteinfall auf den Sensor 30 bzw. alternativ auch bei mechanischer Aktivierung durch den benutzenden Zahnarzt ein Kalibrierprogramm auslöst. Das Kalibrierprogramm regelt eine Nenn-Lichtleistung der Lichtquelle des Handstücks 14, die ebenfalls der Aufnahme 20 benachbart ist, auf einen Nennwert, so dass die Lichtabgabe im Betrieb stets in gleicher Weise erfolgt.

In einer weiteren alternativen Ausgestaltung ist es vorgesehen, dass das Kalibrierprogramm jedes mal automatisch gestartet wird, wenn das Handstück 14 in die Basisstation 12 eingelegt wird. Die Anpassung der Lichtleistung an den Sollwert erfolgt dann automatisch innerhalb der Regelreserve.

Wenn die Regelreserve der Lichtleistung nahezu aufgebraucht ist, erfolgt bevorzugt eine Nachricht an den Zahnarzt oder sonstigen Nutzer, mit welcher dieser veranlasst werden sollte, den Lichtleitstab zu reinigen oder zu ersetzen.

Es versteht sich, dass die erfindungsgemäße Lösung eines Lichthärtgeräts mit integriertem Spiegel nicht auf stiftförmige Handstücke beschränkt ist; vielmehr können in gleicher Weise auf pistolenförmige Lichthärtgeräte, wie sie weit verbreitet sind, entsprechend erfindungsgemäß bestückt sein.

Diese Vertiefung 26 bietet dann eine Ausrichthilfe für die Ausrichtung des Lichthärtgeräts gegenüber dem Spiegel 28, ebenso wie die Vertiefung 26 gemäß Fig. 1 insofern eine Ausrichthilfe bietet.

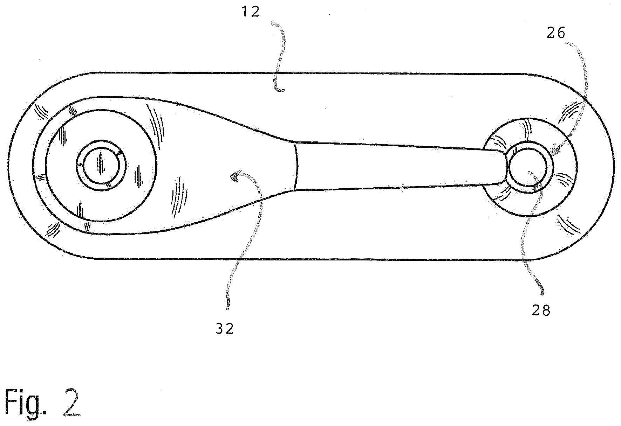

Aus Fig. 2 ist die Basisstation 12 in der Draufsicht ersichtlich. Die Basisstation 12 weist praktisch eine Art Ladeschale 32 für das Handstück 14 auf. Es ist der freiliegende Spiegel 28 ersichtlich, der am Boden der Vertiefung 26 angebracht ist. Da die Vertiefung 26 abgerundet ist, lässt sich der Spiegel relativ leicht sauberhalten, und die räumliche Fixierung, also die Ausrichthilfe erfolgt zwischen der Außenkontur des Handstücks 14 und der Ladeschale 32 der Basisstation 12.

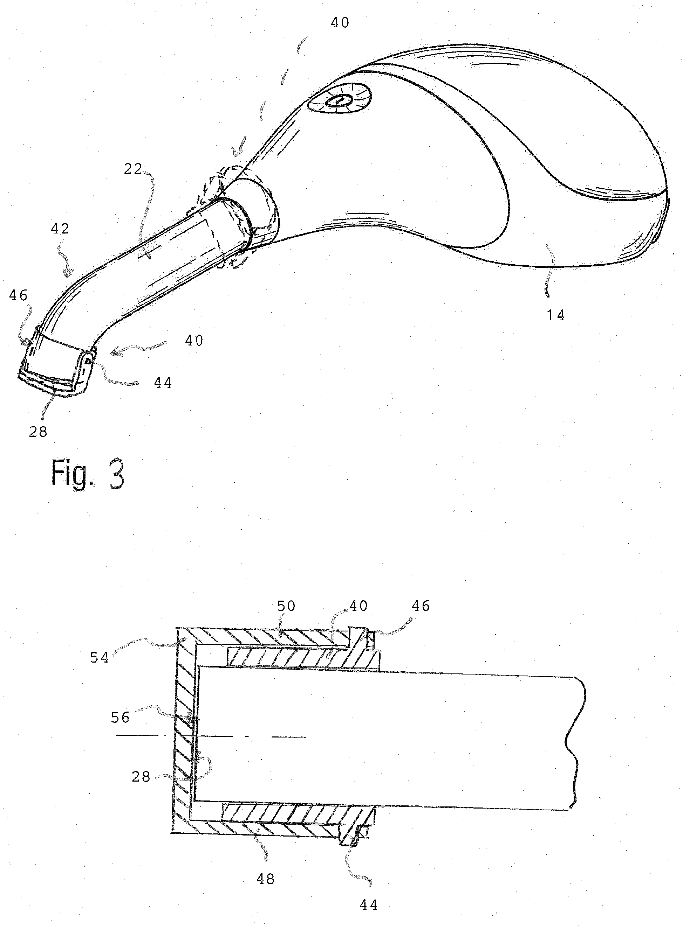

Aus Fig. 3 ist eine modifizierte Ausgestaltung des Lichthärtgeräts ersichtlich, welche nicht Teil der vorliegenden Erfindung ist. Bei dieser Ausführungsform ist der Spiegel 28 am Handstück 14 selbst angebracht. Es ist eine Schiebehülse 40 vorgesehen, die mit ihrem Innendurchmesser zum Außenumfang des Lichtleitstabs 22 passt. Die Schiebehülse 40 besteht aus einem Elastomer und haftet unter einer gewissen Vorspannung auf dem Lichtleitstab. Aufgrund ihrer Elastizität lässt sie sich auch über die Krümmung 42 des Lichtleitstabs hinwegschieben. Weiter unten in Fig. 3 ist der Aufbau im Schnitt dargestellt.

Die Schiebehülse 40 trägt seitlich zwei Lagerzapfen 44 und 46. Die Lagerzapfen werden von Halterarmen 48 und 50 einer Spiegellagerung 54 übergriffen und sind an diesen schwenkbar. Die Spiegellagerung 54 trägt den Spiegel 28, dessen Durchmesser größer als der Durchmesser der Lichtaustrittsfläche 56 des Lichtleitstabs 22 ist. Diese Elemente bieten insofern einen Spiegelaufsatz.

Die Spiegellagerung 54 kann durch Schwenken um die Zapfen 44 und 46 in eine seitliche Position, aber auch die in der Figur dargestellte Kalibrierposition verschwenkt werden. In dieser liegt der Spiegel 28 an der Lichtaustrittsfläche 56 an, die insofern eine Ausrichtshilfe bildet.

Im Betrieb wird nun die Schiebehülse 40 zunächst soweit nach Vorne geschoben, dass ein Verschwenken der Spiegellagerung 54 möglich ist. Dann wird sie erneut zurückgeschoben, so dass der Spiegel 28 auf der Lichtaustrittsfläche 56 aufliegt. In diesem - Zustand wird der Kalibriermodus eingeschaltet und die Kalibrierung wird in der oben beschriebenen Weise vorgenommen.

Nachdem die Kalibrierung erfolgt ist, wird die Schiebehülse 40 entweder kurzerhand abgezogen, oder sie wird in die Fig. 3 gestrichelt dargestellte rückwärtige Position geschoben, natürlich nachdem die Spiegellagerung 54 in die Seitwärtsposition verschwenkt ist.

In der rückwärtigen Position der Spiegellagerung 54 und der Schiebehülse 40 liegt der Lichtleitstab 22 im übrigen frei, und es ergibt sich keine Behinderung bei der Lichthärtung im Mund des Patienten.

In einer weiteren Ausführungsform ist die Spiegellagerung mit an sich bekannten Blendschutzklappen kombiniert, die bei nach außen verschwenkter Spiegellagerung gleichzeitig einen Blendschutz ermöglichen.

In einer weiteren Ausführungsform ist das Spiegelement aus Klebefolie ausgebildet, die auf die Lichtaustrittsfläche 56 aufgeklebt wird und nach Gebrauch verworfen wird.

In einer weiteren Ausführungsform ist es vorgesehen, die Lichtquelle in dem Handstück an das vordere Ende eines Stabes zu verlagern, der dann nicht als Lichtleitstab, sondern als Spannungsversorgungs- und Kühlstab ausgebildet ist. Auch der zugehörige Sensor 30 ist dann an dem vorderen Ende des Handstücks angeordnet, und zwar neben der Lichtquelle, bevorzugt radial außerhalb dieser.

## Patentansprüche

1. Lichthärtgerät für das Härten von Dentalmaterialien, mit einem Handstück (14) und einer Basisstation (12), wobei das Handstück (14) eine Lichtquelle, einen Lichtleitstab (22),der insbesondere austauschbar und lösbar an dem Handstück (14) befestigt ist, sowie einen lichterfassenden Sensor (30) aufweist, der sich im Handstück (14) befindet, wobei das Licht¬härtgerät (10) eine Steuervorrichtung (18) mit mindestens einem Härtprogramm für das Polymerisieren des Dentalmaterials aufweist,
ein Spiegel (28) mit vorgegebenem Reflexionsparameter mit einem Reflektionswert von mehr als 90% vorgesehen ist, **dadurch gekennzeichnet, dass** der Spiegel (28) als Messspiegel in der Basisstation (12) des Lichthärtgeräts (10)integriert ist,
die Basisstation (12) des Lichthärtgeräts (10) eine Ablageposition für das Handstück (14) aufweist, in welcher das Handstück (14) in oder an einer vorgegebenen Urposition aufnehmbar ist, wobei ein Lichtaustrittsende (24) des Lichtleitstabs 22 in eine Vertiefung (26) der Basisstation (12) einfügbar ist, wobei der Spiegel in der Vertiefung (26) der Basisstation (12) angeordnet ist, wobei das Lichthärtgerät (10) eine durch die Vertiefung (26) ausgebildete Ausrichthilfe aufweist, mit welcher die optische Achse des Lichtaustrittsendes (24) des Lichtleitstabs (22) lotrecht zu der Reflektionsfläche des Spiegels (28) ausrichtbar ist, und
dass die Steuervorrichtung (18) ein Kalibrierprogramm aufweist, das mindestens automatisch und/oder per Benutzereingriff auslösbar ist, wenn das Lichthärtgerät (10) mit dem lichtaustrittsseitigen Ende (24) des Lichtleitstabs (22) auf den Spiegel (28) gerichtet ist und in der Ablageposition der Basisstation (12) abgelegt wird, wobei das Kalibrierprogramm bei einem bestimmten Lichteinfall auf den Sensor (30) oder bei mechanischer Aktivierung durch den Benutzer auslösbar ist.

2. Lichthärtgerät nach einem der vorhergehenden Ansprüche, **dadurch gekennzeichnet, dass** der Sensor (30) und die Lichtquelle einem Lichteintrittsende des Lichtleitstabs (22) benachbart angeordnet sind.

3. Lichthärtgerät nach Anspruch 1, **dadurch gekennzeichnet, dass** die optische Achse des Lichtaustrittsendes (24) des Lichtleitstabs (22) lotrecht mit einer Abweichung von weniger als 5° zu der Reflektionsfläche des Spiegels (28) ausrichtbar ist.

4. Lichthärtgerät nach einem der vorhergehenden Ansprüche, **dadurch gekennzeichnet, dass** das Kalibrierprogramm die Nenn-Lichtleistung der Lichtquelle basierend auf dem Messergebnis des Sensors (30) einstellt.

5. Lichthärtgerät nach einem der vorhergehenden Ansprüche, **dadurch gekennzeichnet, dass** der Spiegel (28) als Messspiegel am lichtaustrittsseitigen Ende (24) des Lichthärtgeräts (10) parallel zum Lichtaustrittsende des Lichtleitstabs (22) und von diesem um weniges, insbesondere um weniger als 5mm beabstandet, ausgerichtet ist.

6. Lichthärtgerät nach einem der vorhergehenden Ansprüche, **dadurch gekennzeichnet, dass** das Handstück (14) einen Präsenssensor für den Lichtleitstab (22) aufweist, mit welchem feststellbar ist, ob ein Lichtleitstab (22) an dem Handstück (14) eingesteckt oder nicht eingesteckt, und dass die Steuervorrichtung (18) automatisch das Eigenkalibrationsprogramm durchführt oder zu diesem auffordert, wenn der Präsenssensor den Wechsel des Lichtleitstabs (22) an die Steuervorrichtung (18) gemeldet hat.

7. Lichthärtgerät nach einem der vorhergehenden Ansprüche, **dadurch gekennzeichnet, dass** die Steuervorrichtung (18) an eine Anzeigevorrichtung angeschlossen ist, die den Betriebszustand des Lichthärtgeräts (10) anzeigt.

8. Lichthärtgerät nach Anspruch 7, **dadurch gekennzeichnet, dass** Anzeigevorrichtung die Durchführung des Kalibrationsprogramms anzeigt und den Benutzer auf die Sperre der normalen Betriebsfunktion des Lichthärtgeräts (10) während des Kalibration hinweist.

9. Lichthärtgerät nach Anspruch 7 oder 8, **dadurch gekennzeichnet, dass** die Anzeigevorrichtung die von dem optischen Sensor (30) gemessene und für die Kalibrierung verwendete Lichtleistung als numerischen Wert angibt.

10. Lichthärtgerät nach einem der vorhergehenden Ansprüche, **dadurch gekennzeichnet, dass** das Eigenkalibrationsprogramm der Steuervorrichtung (18) den Lichthärtezyklus bei schwindender und dementsprechend geringer gemessener Leistung der Lichtquellen proportional verlängert.

11. Lichthärtgerät Anspruch 10, **dadurch gekennzeichnet, dass** das Eigenkalibrationsprogramm der Steuervorrichtung (18) den Lichthärtezyklus bei schwindender und dementsprechend geringer gemessener Leistung der Lichtquellen um bis zu 20% verlängert und dass die Belichtungszeit automatisch verlängert wird, also zum Beispiel von 5 Sekunden auf 6 Sekunden.

12. Lichthärtgerät nach einem der Ansprüche 1 und 3 bis 11, **dadurch gekennzeichnet, dass** die Ausrichthilfe als Buchse ausgebildet ist, die den Spiegel (28) umgibt und seitlich abschirmt und sich vor diesem erstreckt und einen zu dem lichtaustrittsseitigen Ende (24) des Lichtleitstabs (22) passenden Innendurchmesser aufweist.

## Claims

1. A light curing apparatus for the curing of dental materials, having a hand pierce (14) and a base station (12), wherein the hand pierce (14) comprises a light source, a light-guiding rod (22), which especially is removably and releasably attached to the hand pierce (14), as well as a light capturing sensor (30) that is located in the hand pierce (14), the light curing apparatus (10) comprising a control device (18) having at least one curing program for the polymerization of the dental material,
a mirror (28) having predeterminded reflexion parameter with a reflexion value of more than 90% is provided, **characterized in that** the mirror (28) is incorporated into the base station (12) of the light curing apparatus (10) as a measuring mirror,
the base station (12) of the light curing apparatus (10) comprises a deposit position for the hand pierce (14), from which the hand pierce (14) may be taken in or at a predetermined original position, wherein a light outlet end (24) of the light-guiding rod 22 is insertable into a recess (26) of the base station (12), wherein the mirror is disposed In the recess (26) of the base station (12), wherein the light curing apparatus (10) comprises an alignment aid formed by the recess (26), by which the optical axis of the light outlet end (24) of the light-guiding rod (22) is orientable perpendicularly to the reflexion surface of the mirror (28), and
**in that** the control device (18) comprises a calibration program, which can be initiated at least automatically and/or via user Intervention, when the light curing apparatus (10) is oriented towards the mirror (28) with the light outlet sided end (24) of the light-guiding rod (22) and/or is deposited In the deposit position of the base station (12), wherein the calibration program can be initiated under specified incidence of light onto the sensor (30) or can be initiated upon mechanical activation by the user.

2. The light curing apparatus according to one of the preceding Claims, **characterized in that** the sensor (30) and the light source are disposed adjacent to a light inlet end of the light-guiding rod (22).

3. The light curing apparatus according to Claim 1, **characterized in that** the optical axis of the light outlet end (24) of the light-guiding rod (22) is perpendicularly orientable, having a deviation of less than 5° in relation to the reflexion surface of the mirror (28).

4. The light curing apparatus according to one of the preceding Claims, **characterized In that** the calibration program sets the nominal power of the light source based on the measuring result of the sensor (30).

5. The light curing apparatus according to one of the preceding Claims, **characterized in that** the mirror (28) is oriented as a measuring mirror at the light outlet sided end (24) of the light curing apparatus (10) parallel to the light outlet end of the light-guiding rod (22) and is slightly spaced apart therefrom and especially is spaced apart by less than 5 mm.

6. The light curing apparatus according to one of the preceding Claims, **characterized in that** the hand pierce (14) comprises a presence sensor for the light-guiding rod (22), with which it may be detected if a light-guiding rod (22) is Inserted Into the hand pierce (14) or not, and that the control device (18) automatically performs the self-calibration program or asks for it, when the presence sensor has transmitted the change of the light-guiding rod (22) to the control device (18).

7. The light curing apparatus according to one of the preceding Claims, **characterized In that** the control device (18) is connected to a display device, which displays the operating status of the light curing apparatus (10).

8. The light curing apparatus according to Claim 7, **characterized in that** the display device displays execution of the calibration program and indicates disabled normal operation of the light curing apparatus (10) during calibration to the user.

9. The light curing apparatus according to Claim 7 or 8, **characterized in that** the display device indicates the light power measured by the optical sensor (30) and used for calibration as a numerical value.

10. The light curing apparatus according to one of the preceding Claims, **characterized in that** the self-calibration program of the control device (18) proportionally extends the den light curing cycle during decrease of, and accordingly low, measuring power of the light sources,

11. The light curing apparatus according to Claim 10, **characterized in that** the self-calibration program of the control device (18) extends the den light curing cycle during decrease of, and accordingly low, measuring power of the light sources by up to 20%, and **in that** the light exposure time is automatically extended, i.e. for example from 5 s to 6 s.

12. The light curing apparatus according to one of the Claims 1 and 3 to 11, **characterized in that** the alignment aid is formed as a socket that surrounds the mirror (28) and shields the sides and extending In front of the mirror and comprising an internal diameter matching the light outlet sided end (24) of the light-guiding rod (22).

## Revendications

1. Appareil de photopolymérisation pour le durcissement des matériaux dentaires, avec une pièce à main (14) et une station de base (12), où la pièce à main (14) présente une source lumineuse, un guide de lumière (22), qui est en particulier interchangeable et attaché de manière détachable à la pièce à main (14), ainsi qu'un capteur de détection de lumière (30), qui se trouve dans la pièce à main (14), où l'appareil de photopolymérisation (10) possède un dispositif de commande (18) avec au moins un programme de durcissement pour la polymérisation du matériel dentaire,
un miroir (28) avec un paramètre de réflexion prédéfini est fourni avec une valeur de réflexion supérieur à 90%, **caractérisé en ce que** le miroir (28) est intégré comme miroir de mesure dans la station de base (12) de l'appareil de photopolymérisation (10),
la station de base (12) de l'appareil de photopolymérisation (10) dispose d'une position de rangement pour la pièce à main (14), dans lequel la pièce à main (14) peut être reçue dans une position de départ prédéterminé, où une extrémité de la sortie de lumière (24) du guide de lumière (22) peut être introduit dans un renfoncement (26) de la station de base (12), où le miroir est disposé dans le renfoncement (26) de la station de base (12), où l'appareil de photopolymérisation (10) présente un aide à l'alignement formé par le renfoncement (26), avec lequel l'axe optique de l'extrémité de sortie de la lumière (24) du guide de lumière (22) peut être orienté perpendiculairement à la surface de réflexion du miroir (28), et
**que** le dispositif de commande (18) présente un programme d'étalonnage, qui-peut être initié au moins de manière automatique et/ou par l'intervention de l'utilisateur, lorsque l'appareil de photopolymérisation (10) est tourné vers le miroir (28) avec l'extrémité de la sortie de lumière (24) du guide de lumière (22) et est rangé dans la position de rangement de la station de base (12), où le programme d'étalonnage peut être déclenché en cas d'une certaine Incidence de la lumière sur le capteur (30) ou lors d'une activation mécanique par l'utilisateur.

2. Appareil de photopolymérisation selon l'une des revendications précédentes, **caractérisé en ce que** le capteur (30) et la source de lumière sont disposés avoisinant une extrémité d'entrée de lumière du guide de lumière (22).

3. Appareil de photopolymérisation selon la revendication 1, **caractérisé en ce que** l'axe optique de l'extrémité de la sortie de lumière (24) du guide de lumière (22) peut être tourné perpendiculaire avec une déviation de moins de 5° par rapport à la surface de réflexion du miroir (28).

4. Appareil de photopolymérisation selon l'une des revendications précédentes, **caractérisé en ce que** le programme d'étalonnage règle la sortie de lumière nominale de la source lumineuse en fonction du résultat de mesure du capteur (30).

5. Appareil de photopolymérisation selon l'une des revendications précédentes, **caractérisé en ce que** le miroir (28) est aligné comme un miroir de mesure à l'extrémité de la sortie de lumière (24) de l'appareil de photopolymérisation, (10) parallèle à l'extrémité de la sortie de lumière du guide de lumière (22) et à une courte distance, en particulier moins de 5mm, de celui-ci.

6. Appareil de photopolymérisation selon l'une des revendications précédentes, **caractérisé en ce que** la pièce à main (14) présente un capteur de présence pour le guide de lumière (22), avec lequel peut être déterminé si un guide de lumière (22) est inséré ou non inséré sur la pièce à main (14), et que le dispositif de commande (18) effectue automatiquement le programme d'auto-étalonnage ou Invite à effectuer celui-ci quand le capteur de présence a signalé l'échange du guide de lumière (22) au dispositif de commande (18).

7. Appareil de photopolymérisation selon l'une des revendications précédentes, **caractérisée en ce que** le dispositif de commande (18) est raccordé à un dispositif d'affichage indiquant l'état de fonctionnement de l'appareil de photopolymérisation (10).

8. Appareil de photopolymérisation selon la revendication 7, **caractérisé en ce que** le dispositif d'affichage affiche l'exécution du programme d'étalonnage et indique à l'utilisateur le verrouillage de la fonction normale de l'appareil de photopolymérisation (10) pendant l'étalonnage.

9. Appareil de photopolymérisation selon la revendication 7 ou 8, **caractérisé en ce que** le dispositif d'affichage indique la quantité de lumière émise mesurée par le capteur optique (30) et utilisé pour l'étalonnage comme valeur numérique.

10. Appareil de photopolymérisation selon l'une des revendications précédentes, **caractérisé en ce que** le programme d'auto-étalonnage du dispositif de commande (18) prolonge proportionnellement le cycle de photopolymérisation en cas d'une baisse de la puissance lumineuse de la source de lumière et la baisse correspondante de la quantité de celle-ci mesurée.

11. Appareil de photopolymérisation selon la revendication 10, **caractérisé en ce que** le programme d'auto-étalonnage du dispositif de commande (18) prolonge proportionnellement le cycle de photopolymérisation en cas d'une baisse de la puissance lumineuse de la source de lumière jusqu'à 20% et que le temps d'exposition est automatiquement prolongé, par exemple de 5 secondes à 6 secondes.

12. Appareil de photopolymérisation selon l'une des revendications 1 et 3 à 11, **caractérisé en ce que** l'aide à l'alignement est formé comme une douille, qui entoure le miroir (28) et le protège latéralement et s'étend devant lui et présente un diamètre intérieur correspondant à l'extrémité de la sortie de lumière (24) du guide de lumière (22).
